# NOUVEAU FASCICULE DE BREVET EUROPEEN

(11) **EP 1 584 329 B2**
(45) Date de publication et mention de la décision concernant l'opposition: **19.05.2021**
(45) Mention de la délivrance du brevet: 05.11.2014
(21) Numéro de dépôt: 05300250.7
(22) Date de dépôt: 01.04.2005
(51) Int. Cl.: A61Q 5/04, A61K 8/46, A45D 7/02

(54) **Procédé de traitement capillaire et utilisation dudit procédé**
Haarbehandlungsverfahren und dessen Einsatz
Hair treatment method and its use

(30) Priorité: 02.04.2004 FR 0450667
(43) Date de publication de la demande: 12.10.2005
(73) Titulaire: L'Oréal, 75008 Paris (FR)
(72) Inventeur: Fondin, Thomas, 95150 Taverny (FR); Sabbagh, Anne, 92500 Rueil Malmaison (FR)
(74) Mandataire: Caillet, Isabelle

(56) Documents cités:
- JP-A- 2000 139 545
- JP-A- 2002 356 408
- US-A- 4 192 863
- US-A- 4 832 948
- US-A- 5 749 379
- US-A1- 2003 033 677
- DATABASE WPI Section Ch, Week 200414 Derwent Publications Ltd., London, GB; Class A96, AN 2004-137526 XP002308308 -& JP 2004 002459 A (MIRUBON KK) 8 janvier 2004 (2004-01-08)
- DATABASE WPI Section Ch, Week 200170 Derwent Publications Ltd., London, GB; Class D21, AN 2001-609747 XP002307747 & JP 2001 213741 A (HOYU KK) 7 août 2001 (2001-08-07)
- DATABASE WPI Week 200107, Derwent Publications Ltd., London, GB; AN 2001-052903 & JP 2000 256146 A (MIRUBON KK) 19 Septembre 2000

## Description

La présente invention se rapporte à un procédé de traitement des fibres capillaires, ainsi qu'à l'utilisation dudit procédé.

Il est usuel, pour obtenir la déformation permanente des cheveux, de réaliser dans un premier temps l'ouverture des liaisons disulfures de la kératine (cystine) à l'aide d'une composition contenant un agent réducteur, puis après avoir de préférence rincé les cheveux, à reconstituer dans un second temps lesdites liaisons disulfures en appliquant sur les cheveux lissés ou préalablement mis sous tension par des moyens adéquats tels que les bigoudis, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Les compositions réductrices utilisables pour la mise en oeuvre de la première étape de ces procédés contiennent généralement des composés thiolés, tels que l'acide thioglycolique, la cystéine, la cystéamine, l'acide thiolactique et le monothioglycolate de glycérol.

La concentration en agents réducteurs peut être très élevée, souvent jusqu'à 15% en poids de la composition réductrice.

Une telle technique ne donne pas entièrement satisfaction. En effet, cette technique est très efficace pour modifier la forme des cheveux, mais aussi très dégradante pour les fibres capillaires.

Il a en outre été proposé d'élever la température des cheveux, entre l'étape de réduction et l'étape de fixation, au moyen d'un fer chauffant.

Ainsi, la demande de brevet JP 2000 256146 décrit un procédé de déformation permanente des cheveux comprenant l'application d'une composition cosmétique contenant 2 à 11 % d'agents réducteurs et de 0,2 à 4% de dithiodiglycolate de diammonium. L'application de la composition réductrice est suivie du passage d'un fer chauffant entre 60 et 220°C.

Cependant, une telle technique d'utilisation d'un fer chauffant s'accompagne en outre d'une étape de fixation après le passage du fer, ce qui augmente la durée du traitement.

De plus, la forme obtenue est irréversible. Le contraste entre les parties traitées et les racines est alors important au moment de la repousse des cheveux.

Enfin, si le traitement est effectué sur des cheveux colorés, on constate très souvent une forte altération de la couleur due au traitement.

Le but de la présente invention est donc de fournir un procédé de traitement des fibres capillaires qui remédie aux inconvénients de l'art antérieur.

En particulier, la présente invention a pour but de fournir un procédé de traitement des fibres capillaires qui permet de modifier le comportement de la fibre capillaire en limitant son altération, de maîtriser le volume des cheveux et d'améliorer les performances cosmétiques des cheveux, notamment la douceur, la brillance et le démêlage en respectant mieux la couleur des cheveux teints.

Ledit procédé doit permettre en outre aux cheveux de garder un aspect naturel, ce qui limite l'effet racines, c'est-à-dire le contraste entre les parties traitées et les racines.

Enfin, l'invention a pour but de réduire la durée du traitement des fibres capillaires et d'obtenir des effets durables.

La Demanderesse a trouvé qu'il était possible de remédier aux inconvénients de l'art antérieur et de remplir les objectifs précités, en mettant en oeuvre un procédé de traitement des fibres capillaires sans fixation comprenant une étape d'application sur les fibres capillaires d'une composition réductrice, contenant au moins un agent réducteur, le ou les agents réducteurs étant choisis parmi les thiols non aminés et représentant moins de 3% en poids du poids total de la composition réductrice, la composition ne contenant pas d'aminothiols, puis une étape d'élévation de la température des fibres capillaires, au moyen d'un fer chauffant, à une température comprise entre 120°C et 220°C, l'élévation de la température étant effectuée avant ou après le rinçage éventuel des fibres capillaires.

Ainsi, l'invention a pour objet un procédé de traitement des fibres capillaires sans fixation comprenant les étapes suivantes :
- application sur les fibres capillaires d'une composition réductrice sans céramide, contenant au moins un agent réducteur, le ou les agents réducteurs étant choisis parmi les thiols non aminés et représentant moins de 3% en poids du poids total de la composition réductrice, la composition ne contenant pas d 'aminothiols.
- élévation de la température des fibres capillaires, au moyen d'un fer chauffant, à une température comprise entre 120°C et 220°C, l'élévation de la température étant effectuée avant ou après le rinçage éventuel des fibres capillaires.

Sans fixation signifie au sens de la présente invention sans application supplémentaire d'une composition contenant un oxydant chimique tel que par exemple le peroxyde d'hydrogène ou un bromate.

De préférence le pH de la composition réductrice est inférieur ou égal à 9 si la composition ne contient pas d'aminothiols.

De préférence la composition réductrice ne contient pas d'acide dithiodiglycolique ou l'un de ses sels.

De préférence, la composition réductrice est appliquée sur des fibres capillaires humides et propres.

Au sens de la présente invention on entend par aminothiol un thiol présentant dans sa structure au moins un groupement NH.

Le ou les thiols utilisés comme agents réducteurs dans la composition réductrice sont choisis les thiols non aminés tels que l'acide thiolactique et ses esters, comme le monothiolactate de glycérol, l'acide thioglycolique et ses esters, comme le monothioglycolate de glycérol ou de glycol, et le thioglycérol.

Lorsque le thiol possède au moins une fonction acide carboxylique, on peut le cas échéant utiliser ledit thiol sous forme d'un ou plusieurs de ses sels, comme les sels de métaux alcalins ou d'ammonium. On peut ainsi utiliser à titre de thiol du thioglycolate d'ammonium.

. Parmi les thiols non aminés utilisés dans l'invention on peut aussi citer le mélange de thioglycolate d'hydroxy-2 propyle (2/3) et de thioglycolate d'hydroxy-2 méthyl-1 éthyle (67/33) décrit dans la demande de brevet FR-A-2 679 448, l'acide β-mercaptopropionique et ses dérivés, et l'acide thiomalique

La concentration totale en thiols non aminés des compositions réductrices de l'invention est comprise de préférence entre 0,1% et 3% et mieux entre 0,5% et 3%.

Le réglage du pH de la composition peut être obtenu à l'aide d'un agent alcalin, tel que, par exemple, l'ammoniaque, les amines organiques comme la monoéthanolamine, la diéthanolamine, la triéthanolamine, la 1,3-propanediamine et le 2-amino-2-méthylpropanol, un carbonate ou bicarbonate alcalin ou d'ammonium, un carbonate organique tel que le carbonate de guanidine, un hydroxyde alcalin, tel que la soude, ou bien à l'aide d'un agent acidifiant tel que par exemple l'acide chlorhydrique, l'acide acétique, l'acide lactique, l'acide oxalique ou l'acide borique.

La composition réductrice comprend généralement un ou plusieurs solvants cosmétiquement acceptables, choisis de préférence parmi l'eau, les alcools en C₁-C₆, de préférence les alcanols tels que l'éthanol, le propanol et l'isopropanol, les polyols tels que le propylène glycol, le pentanediol et la glycérine, l'alcool benzylique, les éthers de polyols, les esters en C₂-C₆, la N-méthylpyrrolidone (NMP), et les cétones en C₃-C₆.

Dans le but d'améliorer les propriétés des compositions capillaires selon l'invention, la composition réductrice utilisée selon l'invention peut également comprendre un ou plusieurs additifs cosmétiques.

Ce ou ces additifs cosmétiques sont généralement choisis parmi les silicones volatiles ou non, linéaires ou cycliques, les polymères cationiques, non-ioniques, anioniques ou amphotères, les peptides et leurs dérivés, les hydrolysats de protéines, les cires, les agents de gonflement et de pénétration ou permettant de renforcer l'efficacité du réducteur tels que le mélange SiO₂/polydiméthylsiloxane, le diméthylisosorbitol, l'urée et ses dérivés, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les agents opacifiants, les colorants, les filtres solaires, les vitamines ou provitamines, les acides gras, les alcools gras, les huiles minérales, végétales ou synthétiques, ainsi que les parfums et les conservateurs, et leurs mélanges.

Par "polymère cationique", on entend tout polymère contenant des groupements cationiques et/ou des groupements ionisables en groupements cationiques.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, que l'on peut utiliser dans la composition de la présente invention, sont notamment décrits dans les brevets français FR 2 505 348 et FR 2 542 997. Parmi ces polymères, on peut citer :
(1) les homopolymères ou copolymères dérivés d'esters ou d'amides de l'acide acrylique ou méthacrylique ;
(2) les dérivés d'éthers de cellulose comportant des groupements ammonium quaternaire décrits dans le brevet français FR 1 492 597 ;
(3) les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropyl-celluloses greffées notamment avec un sel de méthacryloyléthyl-triméthylammonium, de méthacrylamidopropyl-triméthylammonium ou de diméthyldiallylammonium ; on peut citer par exemple le polyquaternium 10 (nom INCI) ;
(4) les autres polysaccharides cationiques décrits plus particulièrement dans les brevets US 3 589 578 et US 4 031 307 tels que les gommes de guar contenant des groupements cationiques trialkylammonium ;
(5) les polymères constitués de motifs pipérazinyle et de groupes divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français FR 2 162 025 et FR 2 280 361 ;
(6) les polyaminoamides solubles dans l'eau, tels que ceux notamment décrits dans les brevets français FR 2 252 840 et FR 2 368 508 ;
(7) les dérivés de polyaminoamides, par exemple, les polymères acide adipique/dialkylaminohydroxyalkyldialkylène-triamine dans lesquels le groupe alkyle comporte de 1 à 4 atomes de carbone et désigne de préférence un groupe méthyle, éthyle, propyle, et le groupe alkylène comporte de 1 à 4 atomes de carbone, et désigne de préférence le groupe éthylène. De tels polymères sont notamment décrits dans le brevet français FR 1 583 363.
(8) les polymères obtenus par réaction d'une polyalkylène-polyamine comportant deux groupements amine primaire et au moins un groupement amine secondaire, avec un acide dicarboxylique choisi parmi l'acide diglycolique et les acides dicarboxyliques aliphatiques saturés ayant de 3 à 8 atomes de carbone. Le rapport molaire entre la polyalkylène-polyamine et l'acide dicarboxylique étant compris entre 0,8 : 1 et 1,4 : 1 ; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains US 3 227 615 et US 2 961 347 ;
(9) les cyclopolymères d'alkyldiallylamine ou de dialkyldiallylammonium tels que l'homopolymère de chlorure de diméthyldiallylammonium et les copolymères de chlorure de diallyldiméthylammonium et d'acrylamide ;
(10) les polymères de diammonium quaternaire présentant une masse moléculaire moyenne en nombre généralement comprise entre 1000 et 100000, tels que, ceux décrits, par exemple, dans les brevets français FR 2 320 330, 2 270 846, 2 316 271, 2 336 434 et 2 413 907 et les brevets US 2 273 780, 2 375 853, 2 388 614, 2 454 547 , 3 206.462, 2 261 002, 2 271 378 , 3.874.870, 4.001.432, 3 929 990, 3 966 904, 4 005 193, 4 025 617, 4 025 627, 4 025 653, 4 026 945 et 4 027 020 ; on peut citer par exemple le chlorure d'hexadiméthrine (nom INCI), commercialisé par la société CHIMEX sous la référence MEXOMERE PO ;
(11) les polymères de polyammonium quaternaire tels que ceux notamment décrits dans la demande de brevet EP-A-122 324 ;
(12) les polymères quaternaires de vinylpyrrolidone et de vinylimidazole tels que, par exemple, les produits commercialisés sous les dénominations Luviquat® FC 905, FC 550 et FC 370 par la société B.A.S.F;
(13) les polyamines comme le Polyquart® H vendu par HENKEL, référencées sous le nom de " POLYETHYLENEGLYCOL (15) TALLOW POLYAMINE " dans le dictionnaire CTFA ;
(14) les polymères réticulés de sels de méthacryloyloxyalkyl(C₁-C₄) trialkyl(C₁-C₄)ammonium tels que ceux commercialisés sous le nom de SALCARE® SC 92, SALCARE® SC 95 et SALCARE® SC 96 par la Société ALLIED COLLOIDS ; et leurs mélanges.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

De préférence, les polymères cationiques sont choisis parmi le chlorure d'hexadiméthrine et les homo ou copolymères de chlorure de diméthyldiallylammonium.

Comme expliqué précédemment, le ou les actifs cosmétiques peuvent être également choisis parmi les silicones.

Comme silicones utilisables comme actifs cosmétiques dans le procédé selon l'invention, on peut citer les polydiméthylsiloxanes, les polyorganosiloxanes quaternisés tels que ceux décrits dans la demande de brevet français FR 2 535 730, les polyorganosiloxanes à groupements aminoalkyles modifiés par des groupements alcoxycarbonylalkyles tels que ceux décrits dans le brevet US n° 4,749,732, des polyorganosiloxanes tels que le copolymère polydiméthylsiloxane- polyoxyalkyle du type diméthicone copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne décrits dans le brevet britannique GB 2 197 352, des polysiloxanes organo modifiés par des groupements mercapto ou mercaptoalkyles tels que ceux décrits dans le brevet français FR 1 530 369 et dans la demande de brevet européen EP 295 780.

Par ailleurs, le ou les actifs cosmétiques peuvent être également choisis parmi les acides gras et les alcools gras.

Comme acides gras utilisables comme actifs dans le procédé selon l'invention, on peut notamment citer les acides carboxyliques en C₈-C₃₀, tels que l'acide palmitique, l'acide oléique, l'acide linoléique, l'acide myristique, l'acide stéarique, l'acide laurique, et leurs mélanges.

Comme alcools gras utilisables dans la présente invention, on peut notamment citer les alcools en C₈-C₃₀ comme, par exemple, les alcools palmitylique, oléylique, linoléylique, myristylique, stéarylique et laurylique.

La composition réductrice utilisée dans le procédé selon l'invention peut se présenter sous la forme d'une lotion, épaissie ou non, d'une crème, d'un gel, ou d'une mousse.

L'application de la composition réductrice telle que définie précédemment constitue donc la première étape du procédé selon l'invention.

Après l'application de la composition réductrice, on peut laisser poser ladite composition, généralement pendant 5 à 60 minutes, de préférence 5 à 30 minutes, éventuellement sous casque.

Comme expliqué précédemment, le procédé selon l'invention comprend, après l'étape d'application de la composition réductrice, une éventuelle étape de rinçage puis une étape d'élévation de la température des fibres capillaires, au moyen d'un fer chauffant, à une température au moins égale à 60°C.

Au sens de la présente invention, on entend par fer un dispositif de chauffage des fibres capillaires par contact.

L'extrémité du fer venant en contact avec les cheveux peut avoir différentes formes. Elle peut notamment présenter une surface plane ; on parle dans ce cas de fer plat. Elle peut également présenter une surface arrondie ; on parle dans ce cas de fer rond.

L'application du fer peut se faire par touches séparées successives de quelques secondes, ou par déplacement ou glissement progressif le long des mèches.

A titre d'exemple de fer utilisable dans le procédé selon l'invention, on peut citer tous types de fer plats ou ronds et, en particulier, de manière non limitative, ceux décrits dans les brevets US 4 103 145, US 4 308 878, US 5 983 903, US 5 957 140, US 5 494 058 et US 5 046 516.

De préférence, la température des fibres capillaires est élevée jusqu'à une température comprise entre 60°C et 250°C, mieux entre 120°C et 220°C.

Selon un mode de réalisation préféré, les fibres capillaires ne sont pas rincées avant l'étape d'élévation de la température.

Le procédé selon l'invention peut également comprendre une étape supplémentaire de pré-séchage partiel des fibres capillaires avant l'étape d'élévation de la température, de manière à éviter d'importants dégagements de vapeurs qui pourraient brûler les mains du coiffeur et le cuir chevelu du sujet. Cette étape de pré-séchage peut se faire par exemple au moyen d'un séchoir, d'un casque, ou bien encore par séchage libre.

La présente invention a également pour objet l'utilisation du procédé tel que décrit précédemment pour modifier de manière durable la forme de la chevelure avec une faible dégradation de la couleur des cheveux et/ou avec une faible dégradation des fibres capillaires.

La présente invention est illustrée par l'exemple suivant.

### Exemple

On met en œuvre le procédé de traitement des fibres capillaires selon l'invention en utilisant une composition réductrice.

La composition réductrice testée est la suivante.

**Composition réductrice**

| | |
|---|---|
| Acide thioglycolique | 1,1 g |
| MEXOMERE PO | 2.5 g |
| 2-amino-2-méthyl-1-propanol | qs pH 9 |
| Eau déminéralisée | qsp 100 g |

Les essais sont réalisés sur des cheveux colorés naturellement frisés.

La composition réductrice telle que décrite précédemment est appliquée sur la tête. On laisse poser la composition pendant 5 minutes

On effectue ensuite un pré-séchage partiel des cheveux au moyen d'un sèche-cheveux.

Puis on passe un fer plat chauffé à 180°C.

On constate en final un bon toucher de la fibre, une maîtrise du volume, un bon respect de la couleur et une bonne rémanence des effets dans le temps.

## Revendications

1. Procédé de traitement des fibres capillaires, sans application supplémentaire d'une composition contenant un oxydant chimique, **caractérisé en ce qu'**il comprend les étapes suivantes :
- application sur les fibres capillaires d'une composition réductrice sans céramide contenant au moins un agent réducteur, le ou les agents réducteurs étant choisis parmi les thiols non aminés et représentant moins de 3% en poids du poids total de la composition réductrice, la composition ne contenant pas d'aminothiols,
- élévation de la température des fibres capillaires, au moyen d'un fer chauffant, à une température comprise entre 120 °C et 220 °C, l'élévation de la température étant effectuée avant ou après le rinçage éventuel des fibres capillaires.

2. Procédé selon la revendication 1, **caractérisé en ce que** le pH est égal ou inférieur à 9.

3. Procédé selon les revendications 1 ou 2, **caractérisé en ce que** la composition réductrice ne contient pas d'acide dithiodiglycolique ou l'un de ses sels.

4. Procédé selon l'une quelconque des revendications précédentes, **caractérisé en ce que** la composition réductrice est appliquée sur des fibres capillaires humides et propres.

5. Procédé selon l'une quelconque des revendications précédentes, **caractérisé en ce que**, après l'application de la composition réductrice, on laisse poser ladite composition réductrice.

6. Procédé selon l'une quelconque des revendications 1 à 3, **caractérisé en ce que** les fibres capillaires ne sont pas rincées avant l'étape d'élévation de la température.

7. Procédé selon l'une quelconque des revendications 1 à 6, **caractérisé en ce qu'**il comprend une étape de pré-séchage partiel des fibres capillaires avant l'étape d'élévation de la température.

8. Procédé selon l'une quelconque des revendications précédentes, **caractérisé en ce que** le ou les thiols non aminés sont choisis parmi l'acide thiolactique et ses esters, l'acide thioglycolique et ses esters, et le thioglycérol.

9. Procédé selon l'une quelconque des revendications précédentes, **caractérisé en ce que** le ou les thiols sont utilisés sous forme de sels.

10. Procédé selon l'une quelconque des revendications précédentes, **caractérisé en ce que** la composition réductrice comprend un ou plusieurs solvants choisis parmi l'eau, les alcools en C₁-C₆, de préférence les alcanols tels que l'éthanol, le propanol et l'isopropanol, les polyols tels que le propylène glycol, le pentanediol et la glycérine, l'alcool benzylique, les éthers de polyols, les esters en C₂-C₆, la N-méthylpyrrolidone (NMP), et les cétones en C₃-C₆.

11. Procédé selon l'une quelconque des revendications précédentes, **caractérisé en ce que** là composition réductrice comprend un ou plusieurs additifs cosmétiques choisis parmi les silicones volatiles ou non, linéaires ou cycliques, les polymères cationiques, non-ioniques, anioniques ou amphotères, les peptides et leurs dérivés, les hydrolysats de protéines, les cires, les agents de gonflement et de pénétration ou permettant de renforcer l'efficacité du réducteur tels que le mélange SiO₂/polydiméthylsiloxane, le diméthylisosorbitol, l'urée et ses dérivés, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les agents opacifiants, les colorants, les filtres solaires, les vitamines ou provitamines, les acides gras, les alcools gras, les huiles minérales, végétales ou synthétiques, ainsi que les parfums et les conservateurs, et leurs mélanges.

12. Procédé selon la revendication 11, **caractérisé en ce que** les polymères cationiques sont choisis parmi le chlorure d'hexadiméthrine et les homo ou copolymères de chlorure de diméthyldiallylammonium.

13. Procédé selon l'une quelconque des revendications précédentes, **caractérisé en ce que** la composition réductrice se présente sous la forme d'une lotion, épaissie ou non, d'une crème, d'un gel ou d'une mousse.

14. Utilisation du procédé selon l'une quelconque des revendications précédentes pour modifier de manière durable la forme de la chevelure avec une faible dégradation de la couleur des cheveux et/ou avec une faible dégradation des fibres capillaires.

## Patentansprüche

1. Verfahren zur Behandlung von Haarfasern ohne zusätzliche Anwendung einer Zusammensetzung, die ein chemisches Oxidationsmittel enthält, **dadurch gekennzeichnet, dass** es die folgenden Schritte umfasst:
- Aufbringen einer ceramidfreien reduzierenden Zusammensetzung, die mindestens ein Reduktionsmittel enthält, auf die Haarfasern, wobei das Reduktionsmittel bzw. die Reduktionsmittel aus nicht aminierten Thiolen ausgewählt ist bzw. sind und weniger als 3 Gew.-%, bezogen auf das Gesamtgewicht der reduzierenden Zusammensetzung, ausmacht bzw. ausmachen, wobei die Zusammensetzung keine Aminothiole enthält,
- Erhöhen der Temperatur der Haarfasern mit Hilfe eines Heizeisens auf eine Temperatur zwischen 120°C und 220°C, wobei die Temperaturerhöhung vor oder nach fakultativem Spülen der Haarfasern durchgeführt wird.

2. Verfahren nach Anspruch 1, **dadurch gekennzeichnet, dass** der pH-Wert kleiner gleich 9 ist.

3. Verfahren nach Anspruch 1 oder 2, **dadurch gekennzeichnet, dass** die reduzierende Zusammensetzung keine Dithiodiglykolsäure oder ein Salz davon enthält.

4. Verfahren nach einem der vorhergehenden Ansprüche, **dadurch gekennzeichnet, dass** man die reduzierende Zusammensetzung auf feuchte und saubere Haarfasern aufbringt.

5. Verfahren nach einem der vorhergehenden Ansprüche, **dadurch gekennzeichnet, dass** man die reduzierende Zusammensetzung nach dem Aufbringen einwirken lässt.

6. Verfahren nach einem der Ansprüche 1 bis 3, **dadurch gekennzeichnet, dass** man die Haarfasern vor dem Temperaturerhöhungsschritt nicht spült.

7. Verfahren nach einem der Ansprüche 1 bis 6, **dadurch gekennzeichnet, dass** es einen Schritt der teilweisen Vortrocknung der Haarfasern vor dem Temperaturerhöhungsschritt umfasst.

8. Verfahren nach einem der vorhergehenden Ansprüche, **dadurch gekennzeichnet, dass** das nicht aminierte Thiol bzw. die nicht aminierten Thiole aus Thiomilchsäure und Estern davon, Thioglykolsäure und Estern davon und Thioglycerin ausgewählt ist bzw. sind.

9. Verfahren nach einem der vorhergehenden Ansprüche, **dadurch gekennzeichnet, dass** man das Thiol bzw. die Thiole in Form von Salzen verwendet.

10. Verfahren nach einem der vorhergehenden Ansprüche, **dadurch gekennzeichnet, dass** die reduzierende Zusammensetzung ein oder mehrere Lösungsmittel, die aus Wasser, C₁-C₆-Alkoholen, vorzugsweise Alkanolen wie Ethanol, Propanol und Isopropanol, Polyolen wie Propylenglykol, Pentandiol und Glycerin, Benzylalkohol, Polyolethern, C₂-C₆-Estern, N-Methylpyrrolidon (NMP) und C₃-C₆-Ketonen ausgewählt sind, umfasst.

11. Verfahren nach einem der vorhergehenden Ansprüche, **dadurch gekennzeichnet, dass** die reduzierende Zusammensetzung ein oder mehrere kosmetische Additive, die aus linearen oder cyclischen, flüchtigen oder nichtflüchtigen Silikonen, kationischen, nichtionischen, anionischen oder amphoteren Polymeren, Peptiden und Derivaten davon, Proteinhydrolysaten, Wachsen, Quellmitteln, Penetriermitteln, Mitteln zur Verstärkung der Wirksamkeit des Reduktionsmittels wie die SiO₂/Polydimethylsiloxan-Mischung, Dimethylisosorbitol, Harnstoff und Derivaten davon, anionischen, kationischen, nichtionischen, amphoteren oder zwitterionischen Tensiden, Mitteln zur Bekämpfung von Haarverlust, Antischuppenmitteln, natürlichen oder synthetischen, assoziativen oder nicht assoziativen Verdickern, Suspendiermitteln, Sequestriermitteln, Trübungsmitteln, Farbmitteln, Lichtschutzmitteln, Vitaminen oder Provitaminen, Fettsäuren, Fettalkoholen, mineralischen, pflanzlichen oder synthetischen Ölen sowie Duftstoffen und Konservierungsmitteln und Mischungen davon ausgewählt sind, umfasst.

12. Verfahren nach Anspruch 11, **dadurch gekennzeichnet, dass** die kationischen Polymere aus Hexadimethrinchlorid und Homo- oder Copolymeren von Dimethyldiallylammoniumchlorid ausgewählt sind.

13. Verfahren nach einem der vorhergehenden Ansprüche, **dadurch gekennzeichnet, dass** die reduzierende Zusammensetzung in Form einer verdickten oder unverdickten Lotion, einer Creme, eines Gels oder eines Schaums vorliegt.

14. Verwendung des Verfahrens nach einem der vorhergehenden Ansprüche zur dauerhaften Modifizierung der Form des Haars mit geringer Degradation der Farbe der Haare und/oder mit geringer Degradation der Haarfasern.

## Claims

1. Process for treating hair fibres, without additional application of a composition containing a chemical oxidizing agent, **characterized in that** it comprises the following steps:
- applying to the hair fibres a ceramide-free reducing composition containing at least one reducing agent, the reducing agent(s) being chosen from non-amino thiols and representing less than 3% by weight relative to the total weight of the reducing composition, the composition not containing any aminothiols,
- raising the temperature of the hair fibres, by means of a heating iron, to a temperature of between 120°C and 220°C, the raising of the temperature being performed before or after optional rinsing of the hair fibres.

2. Process according to Claim 1, **characterized in that** the pH is less than or equal to 9.

3. Process according to Claim 1 or 2, **characterized in that** the reducing composition does not contain any dithiodiglycolic acid or a salt thereof.

4. Process according to any one of the preceding claims, **characterized in that** the reducing composition is applied to clean, wet hair fibres.

5. Process according to any one of the preceding claims, **characterized in that**, after applying the reducing composition, said reducing composition is left in place.

6. Process according to any one of Claims 1 to 3, **characterized in that** the hair fibres are not rinsed before the temperature raising step.

7. Process according to any one of Claims 1 to 6, **characterized in that** it comprises a step of partial predrying of the hair fibres before the temperature raising step.

8. Process according to any one of the preceding claims, **characterized in that** the non-amino thiol(s) are chosen from thiolactic acid and esters thereof, thioglycolic acid and esters thereof, and thioglycerol.

9. Process according to any one of the preceding claims, **characterized in that** the thiol(s) are used in the form of salts.

10. Process according to any one of the preceding claims, **characterized in that** the reducing composition comprises one or more solvents chosen from water, C₁-C₆ alcohols, preferably alkanols such as ethanol, propanol and isopropanol, polyols such as propylene glycol, pentanediol and glycerol, benzyl alcohol, polyol ethers, C₂-C₆ esters, N-methylpyrrolidone (NMP) and C₃-C₆ ketones.

11. Process according to any one of the preceding claims, **characterized in that** the reducing composition comprises one or more cosmetic additives chosen from linear or cyclic, volatile or non-volatile silicones, cationic, nonionic, anionic or amphoteric polymers, peptides and derivatives thereof, protein hydrolysates, waxes, swelling agents and penetrants or agents for reinforcing the efficacy of the reducing agent such as the SiO₂/polydimethylsiloxane mixture, dimethylisosorbitol, urea and derivatives thereof, anionic, cationic, nonionic, amphoteric or zwitterionic surfactants, hair-loss counteractants, antidandruff agents, natural or synthetic, associative or non-associative thickeners, suspension agents, sequestrants, opacifiers, dyes, sunscreens, vitamins or provitamins, fatty acids, fatty alcohols, mineral, plant or synthetic oils, and also fragrances and preserving agents, and mixtures thereof.

12. Process according to Claim 11, **characterized in that** the cationic polymers are chosen from hexadimethrine chloride and homopolymers or copolymers of dimethyldiallylammonium chloride.

13. Process according to any one of the preceding claims, **characterized in that** the reducing composition is in the form of a thickened or unthickened lotion, a cream, a gel or a mousse.

14. Use of the process according to any one of the preceding claims, for durably modifying the shape of the head of hair with a slight degradation of the colour of the hair and/or with a slight degradation of the hair fibres.
